# NEUE EUROPÄISCHE PATENTSCHRIFT

(11) **EP 0 773 022 B3**
(45) Veröffentlichungstag dieser Patentschrift: **06.02.2013**
(45) Hinweis auf die Patenterteilung: 06.02.2002
(21) Anmeldenummer: 96117235.0
(22) Anmeldetag: 27.10.1996
(51) Int. Cl.: A61K 31/415, A61K 31/16, A61K 31/195

(54) **Pharmazeutische Zubereitung zur Behandlung akuter Rhinitiden, enthaltend Sympthomimeticum und Pantothenol und/oder Pantothensaüre**
Pharmaceutical composition for the treatment of rhinitus, containing sympathomometic and pantothenol and/or pantothenic acid
Composition pharmaceutique pour le traitement de la rhinite, contenant un sympathomimétique et du pantothenol et/ou de l'acide pantothénique

(30) Priorität: 10.11.1995 DE 19541919
(43) Veröffentlichungstag der Anmeldung: 14.05.1997
(73) Patentinhaber: M.C.M. KLOSTERFRAU VERTRIEBSGESELLSCHAFT m.b.H., 50670 Köln (DE)
(72) Erfinder: Greve, Rainer, Dr., 23795 Bad Segeberg (DE); Greve, Harald, Dr., 40545 Düsseldorf (DE)
(74) Vertreter: Von Rohr

(56) Entgegenhaltungen:
- EP-A- 0 366 888
- DE-A- 3 317 530
- US-A- 4 665 069
- US-A- 5 304 574
- BREUNINGER H.: "[Diagnosis, differential diagnosis and treatment of inflammation of the nasal mucosa]. DIAGNOSE, DIFFERENTIAL DIAGNOSE UND THERAPIE DER NASENSCHLEIMHAUT ENTZUNDUNG." TAGLICHE PRAXIS, (1976) 17/2 (305-310). CODEN: TAEGBC, XP002096520
- ERNST A. ET AL: "[The medical therapy of the chronic rhinitis with different etiology]. DIE KONSERVATIVE THERAPIE DER CHRONISCHEN RHINITIS UNTERSCHIEDLICHER GENESE." ATEMWEGS- UND LUNGENKRANKHEITEN, (1994) 20/2 (71-75). ISSN: 0341-3055 CODEN: ATLUDF, XP002096521 Germany

## Beschreibung

Die vorliegende Erfindung betrifft eine pharmazeutische Zubereitung, zur Behandlung akuter Rhinitiden.

Für die Behandlung einer akuten Rhinitis steht eine Vielzahl von alpha-Sympathomimetica zur Verfügung, die aufgrund ihrer vasokonstriktorischen Eigenschaften nach lokaler Anwendung in der Nase zur Nasenschleimhautabschwellung führen. Bei wiederholter Anwendung dieser Substanzen ist jedoch häufig eine Austrocknung mit entzündlichen Irritationen der Nasenschleimhäute festzustellen. Diese Situation führt nicht selten zu einer erhöhten Infektionsgefahr, da die Schleimhäute im ausgetrockneten und entzündeten Zustand nicht mehr ihre Schutz- und Filterfunktionen im vollen Umfang aufrechterhalten können und so die Krankheitserreger ungehinderter in die Atemwege gelangen können.

Der vorliegenden Erfindung liegt die Aufgabe zugrunde, eine pharmazeutische Zubereitung zur Behandlung akuter Rhinitiden bereitzustellen, die die Nachteile bereits bekannter Präparate, insbesondere Austrocknung und entzündliche Irritationen der Nasenschleimhäute, vermeidet.

Diese Aufgabe wird erfindungsgemäß durch eine pharmazeutische Zubereitung nach Anspruch 1 gelöst.

Als Pantothenol wird Dexpanthenol (D(+)-Pantothenylalkohol, D(+)-Pantothenol) bevorzugt.

Der antientzündliche Effekt und die Erhöhung der Infektabwehr von Dexpanthenol sind bekannt (beispielsweise Weber, Deutsche Apotheker Zeitung 123, 1921 (1983)). Dexpanthenol ist auch bereits in Form einer Nasensalbe im Handel (Rote Liste 1995).

Es war aber nicht zu erwarten, daß eine pharmazeutische Zubereitung, die Pantothenol in Kombination mit α-Sympathomimetica mit 2-Imidazolin-Struktur enthält, zur Behandlung akuter Rhinitiden sehr viel besser geeignet Ist als die bekannten Monopräparate und durch einen synergistischen Effekt ein Austrocknen und entzündliche Irritationen der Nasenschleimhäute bei der Anwendung vermeidet.

Als zur topischen Anwendung geeignete Sympathomimetica mit 2-Imidazolinstruktur sind erfindungsgemäß die der "generic names" Oxymetazolin oder Xylometazolin sowie deren physiologisch unbedenkliche Salze (s. Erhart-Ruschig, Arzneimittel Band 2, Therapeutika mit Wirkung auf das periphere Nervensystem, S. 150, Tabelle 6).

Das erfindungsgemäße Kombinationspräparat enthält Pantothenol vorzugsweise In Mengen von 0,2 bis 10 Gew.-%, insbesondere 0,2 bis 5 Gew.-%.

Das Sympatomimericum kann in Mengen von 0,01 bis 0,1 Gew.-%, vorzugsweise 0,01 bis 0,05 Gew.-%, vorliegen. Insbesondere liegt es in der Konzentration 0,05 Gew.-% für Erwachsene und 0,025 Gew.-% für Kinder vor.

Die erfindungsgemäße Zubeéreitung enthält die Komponenten a) zu b1) vorzugsweise im Verhältnis von 1 : 50 bis 1 : 500.

Die pharmazeutischen Zubereitungen können flüssig oder dickflüssig bis halbfest sein. Sie können z. B. als Salben, Cremes oder Gele zum Einbringen in die Nase oder als Lösungen zum Tropfen oder Sprühen vorliegen.

Als Träger für flüssige Darreichungsformen eignen sich insbesondere wäßrige Systeme mit oder ohne Zusatz von Glycerin, Sorbit und anderen Polyolen. Als Trägerstoffe für dickflüssige oder halbfeste pharmazeutische Zubereitungen, wie z.B. Salben, Cremes oder Gelen, eignen sich z.B. Paraffinkohlenwasserstoffe, Vaseline, Wollwachs-produkte und andere pharmazeutisch verwendbare, viskositätserhöhende Grundstoffe, bei hydrophilen Gelen z.B. Wasser, Glycerin oder Sorbit, die mit geeigneten Quellstoffen wie z.B. Polyacrylsäure, Zellulosederivaten, Stärke oder Traganth geliert werden.

Die pharmazeutischen Zubereitungen können neben den Wirk- und Trägersubstanzen und gegebenenfalls vorhandenen Emulgatoren noch andere pharmazeutisch unbedenkliche und mit den Wirkstoffen verträgliche Hilfs- und/oder Zusatzstoffe wie z.B. Füll-, Streck-, Binde-, Netz-, Stabilisierungs-, Färbe-, Puffer- und Riechstoffe enthalten. Weiterhin können mikrobiologisch aktive chemische Verbindungen wie z.B. Konservierungsstoffe oder Antiseptika zur Verbesserung der mikrobiellen Stabilität in den pharmazeutisch üblichen Konzentrationen in den Zubereitungen enthalten sein.

Darüber hinaus können die erfindungsgemäßen Zubereitungen auch noch eine oder mehrere andere pharmakologisch wirksame Substanzen enthalten. Insbesondere können noch weitere Verbindungen mit Pantothensäure-Wirkung in freier Form und/oder in Form von Derivaten enthalten sein.

Die Herstellung der erfindungsgemäßen Zubereitungen erfolgt durch Mischen bzw. Lösen der Wirksubstanzen in der pharmakotogisch wirksamen Konzentration, der Hilfs- und/oder Zusatzstoffe sowie der gegebenenfalls weiteren pharmakologisch wirksamen Substanzen in dem vorgesehenen Trägermedium.

Zum Nachweis der synergistischen Wirkungsqualität der erfindungsgemäßen Zubereitungen wurde in Versuchen jeweils eine Nasenseite mit Xylo- bzw. Oxymetazolinhydrochlorid-Lösung allein und die andere Nasenseite mit einer erfindungsgemäßen Zubereitung in Form einer wäßrigen Lösung von Xylo- bzw. Oxymetazolinhydrochlorid und Dexpanthenol behandelt.

In diesen Versuchsreihen ergab die laufende Rhinoskopie bei den mit der erfindungsgemäßen Zubereitung behandelten Nasenseiten eine deutliche klinische Verbesserung gegenüber den Sympathomimetica allein. Darüber hinaus zeigte sich, daß bei der Anwendung der erfindungsgemäßen Zubereitung im Vergleich zu Xylo- bzw. Oxymetazolinhydrochlorid allein keine Reizwirkung und Austrocknung der Schleimhaut festgestellt werden konnte.

Bei der Verwendung der erfindungsgemäßen Zubereitungen sind aufgrund des bisher vorliegenden wissenschaftlichen Erkenntnismaterials über die einzelnen Wirkstoffe keine gravierenden Nebenwirkungen zu erwarten. In den bisher durchgeführten Versuchen konnten ebenfalls keine Nebenwirkungen beobachtet werden.

In Gutachten der Universitäts-Klinik und Poliklinik für Hals-, Nasen- und Ohrenkrankheiten Hamburg-Eppendorf, die Bestandteile dieser Anmeldung sind, wird der durch erfindungsgemäße Kombinationspräparate erzielte synergistische Effekt anhand klinischer Anwendungsversuche an Patienten mit Rhinitiden belegt.

Die folgenden Ausführungsbeispiele veranschaulichen die Herstellung flüssiger und halbfester erfindungsgemäßer Zubereitungen, sind jedoch keinesfalls beschränkend. Der Durchschnittsfachmann kann anhand der Beispiele ersehen, wie durch Variation der einzelnen Parameter die erfindungsgemäße Zubereitung an die jeweiligen Bedingungen anzupassen sind, ohne dabei jedoch die Erfindung zu verlassen.

### Beispiel 1 (Für Erwachsene)

Zur Herstellung von 100 g einer klaren wäßrigen Lösung werden in einem Glasgefäß mit Rühreinrichtung 5 g Dexpanthenol mit 90 g gereinigtem Wasser übergossen und die Substanz durch gründliches Rühren klar gelöst. Der Lösung werden 0,02 g Benzalkoniumchlorid als Konservierungsstoff zugesetzt und gleichfalls unter Rühren gelöst. Dann wird 0,05 g Oxymetazolinhydrochlorid zugesetzt, mit weiterem Wasser zum Endgewicht von 100,0 g aufgefüllt und homogen gerührt. Gegebenenfalls wird die Lösung über neutrale Zellulosefilter filtriert, in Enghalsflaschen aus Braunglas zu 10 oder 20 ml abgefüllt, welche wahlweise mit einer Tropfpipette oder einer Sprüh-Dosierpumpe ausgerüstet werden können.
Anwendung: 2 bis 3mal täglich einen Tropfen bzw. einen Sprühstoß in jedes Nasenloch geben und aufziehen.

### Beispiel 2 (Für Kinder)

Zur Herstellung von 100 g einer klaren wäßrigen Lösung werden in einem Glasgefäß mit Rühreinrichtung 5 g Dexpanthenol mit 90 g gereinigtem Wasser übergossen und die Substanz durch gründliches Rühren klar gelöst. Der Lösung werden 0,02 g Benzalkoniumchlorid als Konservierungsstoff zugesetzt und gleichfalls unter Rühren gelöst. Dann wird 0,025 g Oxymetazolinhydrochlorid zugesetzt, mit weiterem Wasser zum Endgewicht von 100,0 g aufgefüllt und homogen gerührt. Gegebenenfalls wird die Lösung über neutrale Zellulosefilter filtriet, in Enghalsflaschen aus Braunglas zu 10 oder 20 ml abgefüllt, welche wahlweise mit einer Tropfpipette oder einer Sprüh-Dosierpumpe ausgerüstet werden können.
Anwendung: 2 bis 3mal täglich einen Tropfen bzw. einen Sprühstoß in jedes Nasenloch geben und aufziehen.

### Beispiel 3

Zur Herstellung von 100 g einer klaren, gepufferten wäßrigen Lösung mit verstärkter Pantothensäure-Wirkung werden in einem Glasgefäß mit Rühreinrichtung 7,5 g Dexpanthenol mit 85 g gereinigtem Wasser übergossen und durch gründliche Rühren klar gelöst. Zur Einstellung eines stabilen pH-Wertes von 5,3 werden der Lösung 0,756 g Kaliumdihydrogenphosphat und 0,024 g Natriummonohydrogenphosphat-Dodecahydrat als Puffersubstanzen sowie zur Konservierung 0,02 g Benzalkoniumchlorid zugesetzt und unter Rühren gelöst. Die Lösung wird mit 0,05 g Oxymetazolinhydrochlorid versetzt, weiterem Wasser zum Endgewicht von 100,0 g aufgefüllt und homogen gerührt. Gegebenenfalls wird über neutrale Zellulosefilterfiltriert. Die klare, geruchlose Flüssigkeit wird in Enghalsflaschen aus Braunglas zu 10 oder 20 ml abgefüllt, welche wahlweise mit einer Tropfpipette oder einer Sprüh-Dosierpumpe ausgerüstet werden können.
Anwendung: 2 bis 3mal täglich einen Tropfen oder einen Sprühstoß in jedes Nasenloch geben und aufziehen.

### Beispiel 4

Zur Herstellung von 100 g einer klaren, gepufferten wäßrigen Lösung mit erhöhter Haftfähigkeitwerden 5 g Dexpanthenol sowie 0,02 g Benzalkoniumchlorid als Konservierungsstoff in einem Glasgefäß mit Rühreinrichtung mit 80 g gereinigtem Wasser übergossen und durch Rühren klar gelöst. Zur Einstellung eines stabilen pH-Wertes von 5,3 werden der Lösung 0,756 g Kaliumdihydrogenphosphat und 0,024 g Natriummonohydrogenphosphat-Dodecahydrat zugesetzt und unter Rühren gelöst. Zur Herabsetzung der Oberflächenspannung und Erhöhung der Viskosität werden der Lösung noch 0,5 g Sorbit-Lösung 70 % und 0,5 g Glycerin 85 % zugesetzt und die Mischung mit 0,05 g Oxymetazolinhydrochlorid versetzt sowie mit weiterem Wasser zum Endgewicht von 100,0 g aufgefüllt. Zuletzt wird bis zur Schlierenfreiheit homogen gerührt und gegebenenfalls über neutrale Zellulosefilter filtriert. Die klare, geruchlose Flüssigkeit wird in Enghalsflaschen aus Braunglas zu 10 oder 20 ml abgefüllt, welche wahlweise mit einer Tropfpipette oder einer Sprüh-Dosierpumpe ausgerüstet werden können.
Anwendung: 2 bis 3mal täglich einen Tropfen bzw. einen Sprühstoß in jedes Nasenloch geben und aufziehen.

### Beispiel 5

Zur Herstellung von 100 g einer Lösung nach Beispiel 3, jedoch mit verstärkter Pantothensäure-Wirkung, werden in einem Glasgefäß mit Rühreinrichtung 10 g Dexpanthenol sowie 0,02 g Benzalkoniumchlorid als Konservierungsstoff mit 75 g gereinigtem Wasser übergossen und durch gründliches Rühren klar gelöst. Zur Herabsetzung der Oberflächenspannung und Erhöhung der Viskosität und Haftfähigkeit werden der Lösung noch 0,5 g Sorbit-Lösung und 0,5 g Glycerin 85 % zugesetzt und die Mischung mit 0,05 g Oxymetazolinhydrochlorid versetzt sowie mit weiterem Wasser zum Endgewicht von 100,0 g aufgefüllt. Dann wird bis zur Schlierenfreiheil homogen gerührt und gegebenenfalls über neutrale Zellulosefilter filtriert. Die klare, geruchlose Flüssigkeit wird in Enghalsflaschen aus Braunglas zu 10 oder 20 ml abgefüllt, welche wahlweise mit einer Tropfpipette oder einer Sprüh-Dosierpumpe ausgerüstet werden können.
Anwendung: 2 bis3mal täglich einen Tropfen bzw. einen Sprühstoß In jedes Nasenloch geben und aufziehen

### Beispiel 6

Zur Herstellung von 100 g dickflüssigen Gels läßt man in einem verschließbaren Glasgefäß 0,625 g Polyacrylsäure in 50 g gereinigtem Wasser zu einem Sol quellen. Nach etwa 24 Stunden werden unter intensivem Rühren 0,1 g Ammoniaklösung 10 % eingetragen, wobei Gelbildung einsetzt. In einem zweiten Glasgefäß werden sodann 5 g Dexpanthenol sowie 0,02 g Benzalkoniumchlorid als Konservierungsstoff in 40 g gereinigtem Wasser gelöst und die klare Lösungwiederum unter intensivem Rühren langsam in das Polyacrylatgel eingetragen. Zuletzt wird mit 0,05 g Oxymetazolinhydrochlorid versetzt sowie mit weiterem Wasser zum Endgewicht von 100,0 g aufgefüllt und das Produkt bis zur Homogenität gerührt. Man erhält ein fast klares, farb- und geruchloses dickflüssiges Gel, welches in kleine Tuben zu 5 oder 10 g mit ausgezogener Applikationsspitze abgefüllt wird.
Anwendung: Nach Bedarf 2 bis 3mal täglich eine kleine Portion Gel möglichst hoch in beide Nasenlöcher einbringen und aufziehen.

### Beispiel 7

Zur Herstellung einer cremigen Nasensalbe für Kinder werden in einem Glasgefäß mit Rührstab 0.025 g Oxymetazolinhydrochlorid und 5 g Dexpanthenol sowie zur Konservierung 0,02 g Benzalkoniumchlorid in gereinigtem Wasser zu 30,0 g gelöst. In einer Salben-Reibschale mit Pistill wird die wäßrige Wirkstoff-Lösung in 70 g Woltwachsalkohol-Salbe eingearbeitet und unter kräftigem Rühren fein emulgiert. Man erhält eine weiße, weiche Nasensalbe, welche In kleine Tuben zu 5 oder 10 g mit ausgezogener Applikationsspitze abgefüllt wird.
Anwendung: 2 bis 3mal täglich eine kleine Portion Salbe in jedes Nasenloch geben und nach Erweichung durch die Körpertemperatur aufziehen.

### Beispiel 8

### Behandlung von Rhinitiden mit einer Kombination von Oxymetazolin und Dexpanthenol

Oxymetazolin gehört zur Gruppe der α-Sympathomimetika, die aufgrund ihrer vasokonstriktorischen Eigenschaften nach lokaler Anwendung zur Nasenschleimhautabschwellung bei der Behandlung von Rhinitiden therapeutisch genutzt werden. Als Folge eines "Rebound Effektes" kann es nach wiederholter Anwendung von Oxymetazolin zu einer medikamentös bedingten Rhinitis mit entzündlichen Irritationen an der Nasenschleimhaut führen. Dadurch sind die vielfältigen therapeutischen Anwendungsmöglichkeiten von Oxymetazolin weitgehend eingeschränkt. Es wurde überraschend gefunden, daß bei lokaler Anwendung von Dexpanthenol in Kombination mit Oxymetazolin auf der Nasenschleimhaut bereits nach kürzeren Behandlungszeiten ein klinisch manifester Heilungserfolg verzeichnet wurde.

### Methodik

10 Patienten mit Rhinitiden wurden sowohl mit Oxymetazolin-Nasenspray (0,05%) als auch mit der Kombination von Dexpanthenol-Oxymetazolin-Nasenspray (5,0 + 0,05%) behandelt. Der vasokonstriktorische Effekt von Oxymetazolin konnte bezogen auf die Verbesserung der Symptomatik der Nasenschwellung bei allen Patienten deutlich aufgezeigt werden. Auffallend und überraschend war die Beobachtung, daß die von Oxymetazolin ausgehende Reizwirkung nach Applikation der Kombination nicht angegeben wurde, woraus eine größere Compliance der Patienten resultierte. Der nasenabschwellende Effekt erwies sich nach Behandlung mit der Kombination als klinisch wesentlich deutlicher als erwartet.Dies wurde dadurch untermauert, daß der klinische Effekt nach Behandlung mit der Kombination länger anhielt und gleichzeitig eine deutlich bessere Wirkung erzielt wurde. Aufgrund dieser Ergebnisse eines Therapieversuches wurde die Therapie mit der Monosubstanz meist nach 3-tägiger bis 7-tägiger Therapie abgebrochen, um mit der Kombination bei einem deutlich besserem klinischen Effekt fortgesetzt zu werden.

### SchluBtolgerung

Die Ergebnisse des Therapieversuches deuten auf einen Synergismus der Wirkungen des Vasokonstriktors Oxymetazolin und des Dexpanthenols hin, der bei der Behandlung der Rhinitiden zu einer klinisch eindrucksvollen Besserung führt, der über das Ausmaß der Einzelwirkstoffe deutlich hinausgeht.

### Beispiel 9

### Behandlung von Rhinitiden mit einer Kombination von Xylometazolin und Dexpanthenol

Xylometazolin gehört zur Gruppe der α-Sympathomimetika, die wegen ihrer vasokonstriktorischen Eigenschaften zur lokalen Anwendung bei Nasenschleimhautschwellungen im Rahmen der Behandlung von Rhinitiden therapeutisch vielfach genutzt werden. Als Folge eines "Rebound Effektes" kann nach wiederholter Anwendung von Xylometazolin eine medikamentös bedingte Rhinitis mit entzündlichen Irritationen der Nasenschleimhaut entstehen, wodurch die vielfältigen therapeutischen Anwendungsmöglichkeiten von Xylometazolin weitgehend eingeschränkt werden.

Es wurde überraschend gefunden, daß nach lokaler Anwendung von Xylometazolin in Kombination mit Dexpanthenol bereits nach kürzeren Behandlungszeiten ein klinisch manifester Heilungserfolg auf der Nasenschleimhaut festgestellt werden konnte, der deutlicher war, als bei lokaler Anwendung von Xylometazolin allein.

### Methodik

Im Rahmen der Therapie von Rhinitiden wurden je 12 Patienten mit einem Xylometazolin-Nasenspray (0,05%) und auch einer Kombination aus Dexpanthenol (5%) mit Xylometazolin (0,05%) behandelt. Bei allen Patienten konnte eine deutliche Besserung der Nasenschwellung, entsprechend dem Wirkmechanismus des vasokonstriktorischen Effektes von Xylometazolin, aufgezeigt werden.

Auffallend und überraschend war die Beobachtung, daß die von Xylometazolin ausgehende Reizwirkung nach Applikation der Kombination von Xylometazolin mit Dexpanthenol nicht auftrat, so daß für die Patienten eine bessere Compliance resultierte. Nach Behandlung mit der Kombination erwies sich der nasenabschwellende Effekt als klinisch wesentlich deutlicher im Vergleich zur Behandlung mit Xylometezolin allein. Dieser unerwartete klinische Effekt wurde dadurch untermauert, daß die Wirkung nach Behandlung mit der Kombination länger anhielt und gleichzeitig eine bessere Heilung erzielt wurde. Aufgrund der Ergebnisse eines Therapieversuches wurde die Behandlung mit Xylometazolin alleine meist nach 3- bis 7-tägiger Therapie abgebrochen, um mit der Kombination aus Xylometazolin und Dexpanthenol bei einem deutlich besseren klinischen Effekt fortgesetzt zu werden.

### SchluBfolgerung

Die klinischen Untersuchungen bestätigen einen unerwarteten Synergismus der Wirkungen von Xylometazolin und Dexpanthenols, der bei der Behandlung der Rhinitiden zu einer klinisch deutlicheren Besserung führt, der über das Ausmaß der Einzelwirkstoffe deutlich hinausgeht.

## Patentansprüche

1. Pharmazeutische Zubereitung zur topischen Behandlung akuter Rhinitiden, enthaltend in Kombination und in physiologischer Konzentration
a) ein zur topischen Anwendung geeignetes Sympathomimeticum mit 2-Imidazolin-Struktur oder dessen physiologisch unbedenkliche Salze, wobei das Sympathomimeticum Oxymetazolin oder Xylometazolin ist; und
b1) Pantothenol.

2. Pharmazeutische Zubereitung nach Anspruch 1, **dadurch gekennzeichnet, daß** das Sympathomimeticum Oxymetazolinhydrochlorid ist.

3. Pharmazeutische Zubereitung nach Anspruch 1, **dadurch gekennzeichnet, daß** das Sympathomimeticum Xylometazolinhydrochlorid ist.

4. Pharmazeutische Zubereitung nach einem der Ansprüche 1 bis 3, **dadurch gekennzeichnet, daß** das Pantothenol D(+)-Pantothenol (Dexpanthenol) ist.

5. Pharmazeutische Zubereitung nach einem der Ansprüche 1 bis 4, enthaltend das Sympathomimeticum a) in Mengen von 0,01 bis 0,1 Gew.-%, vorzugsweise 0,01 bis 0,05 Gew.-%.

6. Pharmazeutische Zubereitung nach einem der Ansprüche 1 bis 5, enthaltend die Komponente b1) in Mengen von 0,2 bis 10 Gew.-%, vorzugsweise 0,2 bis 5 Gew.-%.

7. Pharmazeutische Zubereitung nach einem der Ansprüche 1 bis 6, enthaltend die Komponente a) zu b1) im Verhältnis 1 : 50 bis 1 : 500.

8. Pharmazeutische Zubereitung nach einem der Ansprüche 1 bis 7, enthaltend übliche Träger und Zusatzstoffe.

9. Verwendung der pharmazeutischen Zubereitung nach einem der Ansprüche 1 bis 8 zur Herstellung eines Arzneimittels zur topischen Behandlung akuter Rhinitiden.

## Claims

1. Pharmaceutical composition for the topical treatment of acute rhinitis, containing in combination and in physiological concentration
a) a sympathomimetic, suitable for topical application, with a 2-imidazoline structure, or its physiologically harmless salts, wherein the sympathomimetic is oxymetazoline or xylometazoline; and
b1) pantothenol.

2. Pharmaceutical composition according to claim 1, **characterised in that** the sympathomimetic is oxymetazoline hydrochloride.

3. Pharmaceutical composition according to claim 1 **characterised in that** the sympathomimetic is xylometazoline hydrochloride.

4. Pharmaceutical composition according to one of claims 1 to 3, **characterised in that** the pantothenol is D(+)-pantothenol (dexpanthenol).

5. Pharmaceutical composition according to one of claims 1 to 4, containing the sympathomimetic a) in amounts of between 0.01 and 0.1 wt-%, preferably between 0.01 and 0.05 wt-%.

6. Pharmaceutical composition according to one of claims 1 to 5, containing the components b1) and/or b2) in amounts of between 0.2 and 10 wt-%, preferably between 0.2 and 5 wt-%.

7. Pharmaceutical composition according to one of claims 1 to 6, containing the components a) to b1) and/or b2) in a ratio of between 1 : 50 and 1 : 500.

8. Pharmaceutical composition according to one of claims 1 to 7, containing standard carriers and additives.

9. Use of the pharmaceutical composition according to one of claims 1 to 8 to produce a medicine for the topical treatment of acute rhinitis.

## Revendications

1. Préparation pharmaceutique destinée au traitement topique de rhinites aiguës, contenant conjointement et à une concentration physiologique:
a) un sympathomimétique approprié à un usage topique avec une structure de 2-imidazoline ou ses sels physiologiquement acceptables, où le sympathomimétique est l'oxymétazoline ou le xylométazoline; et
b1) du pantothénol.

2. Préparation pharmaceutique selon la revendication 1, **caractérisée en ce que** le sympathomimétique est le chlorhydrate d'oxymétazoline.

3. Préparation pharmaceutique selon la revendication 1, **caractérisée en ce que** le sympathomimétique est le chlorhydrate de xylométazoline.

4. Préparation pharmaceutique selon l'une des revendication 1 à 3, **caractérisée en ce que** le pantothénol est du D(+)-panthothénol (dexpanthénol).

5. Préparation pharmaceutique selon l'une des revendication 1 à 4, contenant le sympathomimétique a) en des quantités de 0,01 à 0,1 % en pois, de préférence de 0,01 à 0,05 % en poids.

6. Préparation pharmaceutique selon l'une des revendications 1 à 5, contenant les composants b1) et/ou b2) en des quantités de 0,2 à 10 % en poids, de préférence de 0,2 à 5 % en poids.

7. Préparation pharmaceutique selon l'une des revendications 1 à 6, contenant les composants a) à b1) et/ou b2) dans le rapport de 1 50 à 1 : 500.

8. Préparation pharmaceutique selon l'une des revendications 1 à 7, contentant des véhicules et des additifs usuels.

9. Utilisation de la préparation pharmaceutique selon l'une des revendication 1 à 8 pour la préparation d'un médicament destiné au traitement topique de rhinites aiguës.
